(19) Europäisches Patentamt
European Patent Office
Office européen des brevets

(11) **EP 4 316 499 B1**

(12) **EUROPEAN PATENT SPECIFICATION**

(45) Date of publication and mention
of the grant of the patent:
**28.01.2026   Bulletin 2026/05**

(21) Application number: **22776005.5**

(22) Date of filing: **21.03.2022**

(51) International Patent Classification (IPC):
*A61K 35/747* [(2015.01)]      *A61K 36/062* [(2006.01)]
*A61K 36/8945* [(2006.01)]    *A61K 36/346* [(2006.01)]
*A61K 36/07* [(2006.01)]       *A61P 35/00* [(2006.01)]
*A23L 33/135* [(2016.01)]      *A23L 33/105* [(2016.01)]
*A23K 10/16* [(2016.01)]       *A23K 10/30* [(2016.01)]
*A23K 10/18* [(2016.01)]

(52) Cooperative Patent Classification (CPC):
(C-Sets available)
**A23K 10/18; A23L 33/135; A61K 35/747;**
**A61P 35/00;** A23V 2002/00      (Cont.)

(86) International application number:
**PCT/KR2022/003890**

(87) International publication number:
**WO 2022/203303 (29.09.2022 Gazette 2022/39)**

(54) **LACTOBACILLUS PLANTARUM GB104 STRAIN AND COMPOSITION COMPRISING SAME FOR PREVENTION OR TREATMENT OF CANCER**

LACTOBACILLUS PLANTARUM GB104 STAMM UND ZUSAMMENSETZUNG DAMIT ZUR PRÄVENTION ODER BEHANDLUNG VON KREBS

SOUCHE DE LACTOBACILLUS PLANTARUM GB104 ET COMPOSITION LA COMPRENANT DESTINÉE À LA PRÉVENTION OU AU TRAITEMENT DU CANCER

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **22.03.2021   KR 20210036881**

(43) Date of publication of application:
**07.02.2024   Bulletin 2024/06**

(73) Proprietor: **GI BIOME**
**Gyeonggi-do 13201 (KR)**

(72) Inventors:
• **JANG, Myung Ho**
  **Seoul 05849 (KR)**
• **YANG, Bo-Gie**
  **Seoul 05849 (KR)**
• **KIM, A-Ram**
  **Namyangju-si Gyeonggi-do 12095 (KR)**

(74) Representative: **HGF**
**HGF Limited**
**4th Floor, 1 City Square**
**Leeds LS1 2AL (GB)**

(56) References cited:
**WO-A1-2019/085521      WO-A1-2021/137603
CN-A- 105 441 357       KR-A- 20020 072 913
KR-A- 20110 046 020     KR-A- 20130 132 398
KR-A- 20140 098 708     KR-A- 20170 060 458
KR-B1- 101 287 120      KR-B1- 101 580 732
KR-B1- 101 919 938      KR-B1- 102 028 744**

- **SUN MENGYING ET AL: "The Effects of Lactobacillus plantarum-12 Crude Exopolysaccharides on the Cell Proliferation and Apoptosis of Human Colon Cancer (HT-29) Cells", vol. 13, no. 2, 26 August 2020 (2020-08-26), New York, NY ; Heidelberg : Springer, pages 413 - 421, XP093182239, ISSN: 1867-1306, Retrieved from the Internet <URL:https://link.springer.com/article/10.1007/s12602-020-09699-8/fulltext.html> DOI: 10.1007/s12602-020-09699-8**

Remarks:
    The file contains technical information submitted after the application was filed and not included in this specification

(52) Cooperative Patent Classification (CPC): (Cont.)

    C-Sets
    A23V 2002/00, A23V 2200/308, A23V 2400/169

Remarks:
    The file contains technical information submitted after the application was filed and not included in this specification

## Description

### Technical Field

The present invention relates to a *Lactobacillus plantarum* GB104 strain (accession number: KCTC 14107BP) and a composition for preventing or treating cancer, comprising the same as an active ingredient.

The invention is defined by the appended claims 1-5.

### Background Art

Worldwide, cancer is considered as one of the major health problems. Among cancers, large intestine cancer is the fourth most common malignant tumor worldwide and the third leading cause of cancer death. Looking at the statistics of large intestine cancer in Korea, although it was not a common disease before the 1970s, the incidence of large intestine cancer is gradually increasing as the diet has become westernized in modern times. Currently, surgical operation is the most effective treatment method for large intestine cancer, like other digestive system cancers. The survival rate from the early stage 1 to stage 3 ranges from 70 to 90%, but the survival rate drops sharply to 15% in the final stage 4.

Although many anticancer agents have been clinically developed for the treatment of large intestine cancer, long-term use thereof causes many side effects. For example, it has been reported that fluorouracil causes neutropenia, stomatitis and diarrhea, irinotecan causes myelosuppression, nausea and alopecia, and oxaliplatin causes paresthesia and renal dysfunction. In this situation, there is an urgent need to create an anticancer substance that can contribute to the improvement of human health, the improvement of the quality of healthy life, and the promotion of human well-being by enabling early prevention and treatment of cancer.

On the other hand, lactic acid bacteria are widely distributed in the oral cavity, intestines, vagina, and feces of humans and animals, and fermented foods such as kimchi, and are closely related to human and animal health. Lactic acid bacteria exhibit various health-promoting effects such as intestinal regulation, suppression of harmful bacteria, immune regulation, blood cholesterol lowering, and anticancer effects.

Currently, Korean Patent Application Publication No. 10-2015-0068061 discloses the anticancer activity of *Lactobacillus plantarum* PNU (KCCM11352P) or *Lactobacillus mesenteroides* PNU (KCCM11353P), and Korean Patent Registration No. 10-1287120 discloses a pharmaceutical composition for treating cancer, containing *Lactobacillus plantarum* DSR CK10 (accession number: KFCC-11433P) or *Lactobacillus plantarum* DSR M2 (accession number: KFCC-11432P) as an active ingredient. However, the effect has not reached the level of commercial success

Korean Patent Application KR20130132398 as well discloses the anticancer activity of Lactobacillus plantarum I-UL4, TL1, RS5, RG14, RG11 and RI11.

Therefore, there is a need for continuous research on a new strain having an excellent anticancer effect.

### Detailed Description of Invention

#### Technical Problem

Accordingly, the present inventors studied to develop a new strain having an excellent anticancer effect, and as a result, it was found that a *Lactobacillus plantarum* GB104 strain (accession number: KCTC 14107BP) exhibited excellent anticancer effect. Based on the above, the present inventors completed the present invention.

#### Solution to Problem

In order to achieve the above object, in one aspect of the present invention, there is provided a pharmaceutical composition for use in preventing or treating cancer, comprising a *Lactobacillus plantarum* GB104 (*L. plantarum* GB104) strain (accession number: KCTC 14107BP) or a culture product thereof as an active ingredient.

In another aspect of the present invention, there is provided a Lactobacillus plantarum GB104 strain (accession number KCTC 14107BP), or a culture product thereof, for use in a method for preventing or treating cancer, the method comprising administering the *Lactobacillus plantarum* GB104 (*L. plantarum* GB104) strain (accession number: KCTC 14107BP) or the culture product thereof to a subject other than a human being.

#### Effects of Invention

The *Lactobacillus plantarum* GB104 strain (accession number: KCTC 14107BP) according to the present invention increases the production of INF-γ and granzyme B and activates immune cells such as cytotoxic T cells and NK cells in a cancer cell line or a mouse model transplanted with a cancer cell line, thereby ultimately exhibiting an inhibitory effect on tumor formation and tumor growth. Therefore, the composition of the present invention comprising a *Lactobacillus plantarum* GB104 strain as an active ingredient can be usefully used for preventing, improving or treating cancer diseases.

**Brief Description of Drawings**

Fig. 1 illustrates the results obtained by confirming the colony forming ability after treatment with a *L. plantarum* GB104 strain or general lactic acid bacteria 1 (*L. Fermentum* GB102) in the human large intestine cancer cell lines HCT116 and HT-29, respectively. Here, (-) is a group not treated with any strain as a negative control group.

Fig. 2 is a graph showing the colony forming ability in percentage (%) relative to a negative control group after treatment with a *L. plantarum* GB104 strain or general lactic acid bacteria 1 (*L. Fermentum* GB102) in the human large intestine cancer cell lines HCT116 and HT-29, respectively. ** P < 0.01

Fig. 3 illustrates the results obtained by confirming the colony forming ability after treatment with a culture solution of a *L. plantarum* GB104 strain or general lactic acid bacteria 1 (*L. Fermentum* GB102) in the human large intestine cancer cell lines HCT116 and HT-29, respectively.

Fig. 4 is a graph showing the colony forming ability in percentage (%) relative to a negative control group after treatment with a culture solution of a *L. plantarum* GB104 strain or general lactic acid bacteria 1 (*L. Fermentum* GB102) in the human large intestine cancer cell lines HCT116 and HT-29, respectively. ** P < 0.01; *** P < 0.001

Fig. 5 is a diagram showing an experimental schedule for confirming the cancer prevention effect of general lactic acid bacteria 1 (*L. Fermentum* GB102), general lactic acid bacteria 2 (*L. Fermentum* GB103) or a *L. plantarum* GB104 strain in a mouse-derived large intestine carcinoma CT-26 animal model.

Fig. 6 is a graph showing the results obtained by measuring the degree of tumor size and growth after administration with general lactic acid bacteria 1 (*L. Fermentum* GB102), general lactic acid bacteria 2 (*L. Fermentum* GB103) and a *L. plantarum* GB104 strain, respectively, in a mouse-derived large intestine carcinoma CT-26 animal model. * P < 0.05

Fig. 7 is a graph showing the results obtained by measuring the degree of tumor size and growth of individual experimental animals from a negative control group and a group treated with a *L. plantarum* GB104 strain in a mouse-derived large intestine carcinoma CT-26 animal model.

Fig. 8 is a diagram showing an experimental schedule for confirming the anticancer activity of general lactic acid bacteria 3 (*L. Helveticus* MG585) or a *L. plantarum* GB104 strain in a mouse-derived large intestine carcinoma CT-26 animal model.

Fig. 9 is a graph showing the results obtained by measuring the degree of tumor size and growth after administration with general lactic acid bacteria 3 (*L. Helveticus* MG585) and a *L. plantarum* GB104 strain, respectively, in a mouse-derived large intestine carcinoma CT-26 animal model. ** P < 0.01

Fig. 10 is a graph showing the results obtained by measuring the degree of tumor size and growth of individual experimental animals from a negative control group and a group treated with a *L. plantarum* GB104 strain in a mouse-derived large intestine carcinoma CT-26 animal model.

Fig. 11 is a graph (left) showing the results obtained by measuring the tumor weight after treatment with a *L. plantarum* GB104 strain in a mouse-derived large intestine carcinoma CT-26 animal model and a photograph (right) showing the tumor size compared to a negative control group. * P < 0.05

Fig. 12 is a diagram showing an experimental schedule for confirming the anticancer activity of a *L. plantarum* GB104 strain in a mouse-derived large intestine carcinoma MC-38 animal model.

Fig. 13 is a graph showing the results obtained by measuring the degree of tumor size and growth after administration with a *L. plantarum* GB104 strain in a mouse-derived large intestine carcinoma MC-38 animal model. ** P < 0.01

Fig. 14 is a graph showing the results obtained by measuring the tumor weight after treatment with a *L. plantarum* GB104 strain in a mouse-derived large intestine carcinoma MC-38 animal model. * P < 0.05

Fig. 15 is a graph showing the results obtained by measuring the degree of tumor size and growth of individual experimental animals from a negative control group and a group treated with a *L. plantarum* GB104 strain in a mouse-derived large intestine carcinoma MC-38 animal model.

Fig. 16 is a diagram showing an experimental schedule for confirming the changes in immune cells in the early stage tumor tissue and peripheral blood after administration with a *L. plantarum* GB104 strain in a mouse-derived large intestine carcinoma MC-38 animal model.

Fig. 17 is a graph showing the results obtained by analyzing the changes in immune cells in the early stage tumor tissue after administration with a *L. plantarum* GB104 strain in a mouse-derived large intestine carcinoma MC-38 animal model. * P < 0.05; **** P < 0.0001

Fig. 18 is a graph showing the results obtained by analyzing the changes in immune cells in the early stage peripheral blood after administration with a *L. plantarum* GB104 strain in a mouse-derived large intestine carcinoma MC-38 animal model. * P < 0.05; ** P < 0.01; *** P < 0.001

Fig. 19 is a diagram showing an experimental schedule for confirming the changes in immune cells in the late stage tumor tissue after administration with general lactic acid bacteria 4 (*L. plantarum* GBCC_f0057) and a *L. plantarum* GB104 strain, respectively, in a mouse-derived large intestine carcinoma MC-38 animal model.

Fig. 20 is a graph showing the results obtained by measuring the degree of tumor size and growth after administration with general lactic acid bacteria 4 (*L. plantarum* GBCC_f0057) and a *L. plantarum* GB104 strain, respectively, in a mouse-derived large intestine carcinoma MC-38 animal model. * P < 0.05

Fig. 21 is a graph showing the results obtained by measuring the degree of tumor size and growth of individual experimental animals from a negative control group and a group treated with a *L. plantarum* GB104 strain in a mouse-derived large intestine carcinoma MC-38 animal model.

Fig. 22 is a graph showing the results obtained by analyzing the changes in immune cells in the late stage tumor tissue after administration with general lactic acid bacteria 4 (*L. plantarum* GBCC_f0057) and a *L. plantarum* GB104 strain, respectively, in a mouse-derived large intestine carcinoma MC-38 animal model. * P < 0.05; ** P < 0.01

## Best Mode for Carrying out the Invention

### Pharmaceutical composition comprising *L. plantarum* GB104 strain or culture product thereof

In one aspect of the present invention, there is provided a pharmaceutical composition for preventing or treating cancer, comprising a *Lactobacillus plantarum* GB104 (*L. plantarum* GB104) strain (accession number: KCTC 14107BP) or a culture product thereof as an active ingredient.

As used herein, the term *"Lactobacillus"* is an aerobic or facultative anaerobic gram-positive microorganism of the genus Bacillus widely distributed in nature, and microorganisms belonging to the genus Lactobacillus include *Lactobacillus plantarum, Lactobacillus sakei,* and the like.

As a result of research to develop a new strain having an excellent anticancer effect, the present inventors selected *Lactobacillus plantarum* GB104 (*L. plantarum* GB104) as an anticancer candidate strain. The strain was deposited with the Korean Collection for Type Cultures, the Korea Research Institute of Bioscience and Biotechnology on January 14, 2020 under the accession number of KCTC 14107BP, for which a new strain patent application was filed as Korean Patent Application No. 10-2020-0186738. The strain is a probiotic strain, which is harmless to the human body and can be used without side effects.

As used herein, the term *"Lactobacillus plantarum* GB104" can be interchangeably described as *L. plantarum* GB104, *Lactobacillus plantarum* GB104, or a *Lactobacillus plantarum* GB104 strain (accession number: KCTC 14107BP).

The *L. plantarum* GB104 strain (accession number: KCTC 14107BP) is the same as described above. In this case, the strain may be a viable cell, a dead cell, or a cytoplasmic fraction obtained by disrupting the strain, and the strain may be preferably a viable cell.

As used herein, the term "culture product" refers to a product obtained by culturing a probiotic strain in a known medium, and the product may or may not include the strain itself. The medium may be selected from known liquid media or solid media. For example, it may be MRS liquid medium, GAM liquid medium, MRS agar medium, GAM agar medium, BL agar medium, but is not limited thereto.

The composition comprises a *L. plantarum* GB104 strain or a culture product thereof, as an active ingredient, in a therapeutically effective amount or in a nutritionally effective concentration based on the total weight of the composition. The composition comprises an active ingredient in an amount of $10^4$ to $10^{16}$ CFU/g, and preferably $10^6$ to $10^{12}$ CFU/g, or comprises a culture product containing an equivalent number of viable cells. In general, for adult patients, viable cells in an amount of $1 \times 10^6$ CFU/g or more, and preferably $1 \times 10^8$ to $1 \times 10^{12}$ CFU/g may be administered once or in several divided doses.

The strain of the present invention can inhibit tumor formation. In addition, the strain activates immune cells.

As used herein, the term "immunity" refers to a mechanism that protects a living organism from a disease by detecting and incapacitating pathogens and tumor cells in the organism. The "immunostimulation" can include all of a series of therapeutic or prophylactic strategies that enhance the body's defense mechanisms against various diseases such as cancer and inflammation.

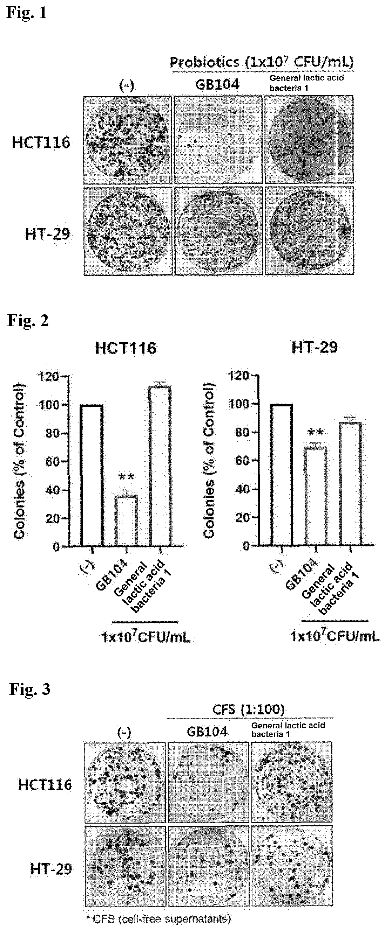

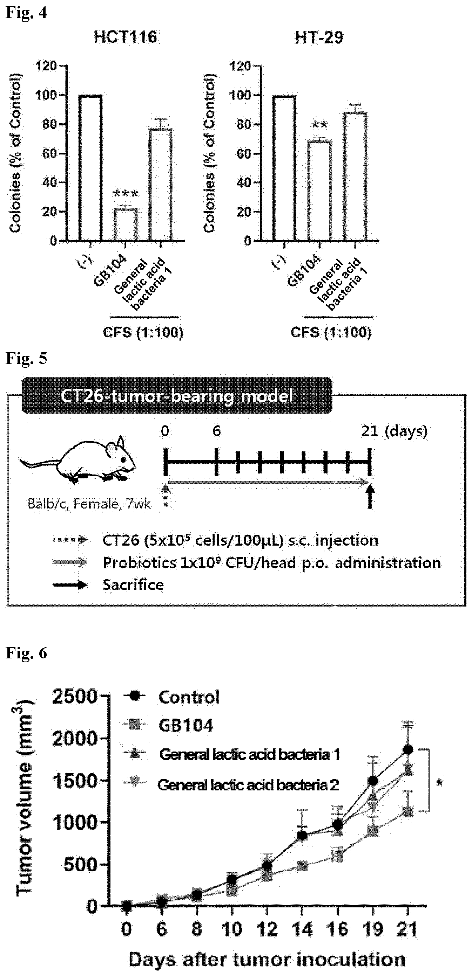

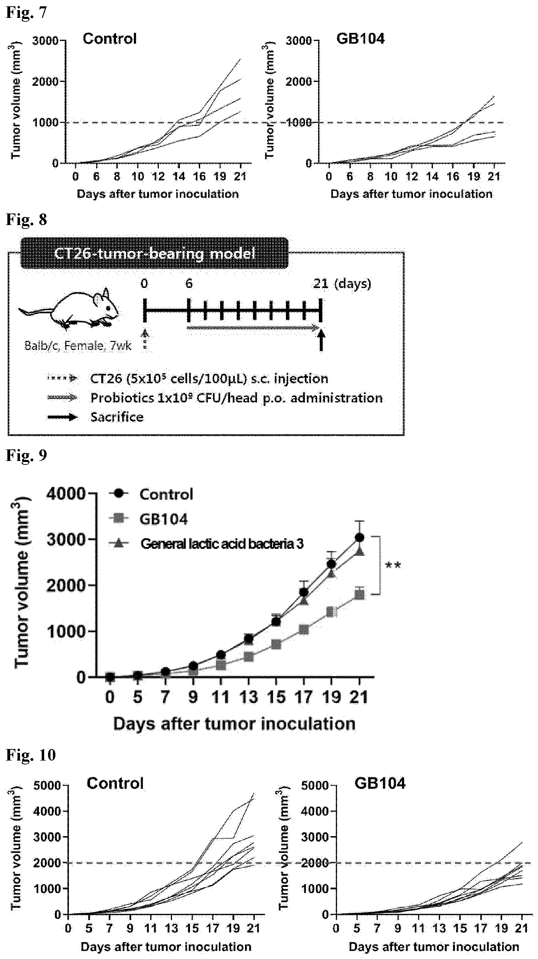

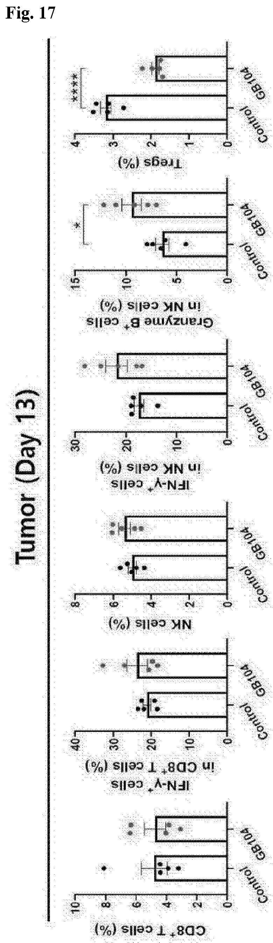

In one embodiment of the present invention, when the large intestine cancer cell line was treated with the strain or a culture solution from which the strain was removed, the formation of tumor cell colonies was significantly reduced compared to the negative control group (Figs. 1 to 4). In addition, as a result of examining the degree of tumor formation by administering the strain from the time when the mouse-derived large intestine cancer cell line was subcutaneously transplanted to the mouse, it was confirmed that tumor formation was inhibited compared to the negative control group (Figs. 5 to 7). In addition, as a result of examining the growth of the tumor by administering a *L. plantarum* GB104 strain to a tumor animal model in which a tumor having a certain size was formed, it was confirmed that the tumor growth rate was significantly reduced compared to the negative control group (Figs. 8 to 22). In this case, the number of cytotoxic T cells and NK cells in tumor tissue and blood was significantly increased, and the distribution of IFN-γ and granzyme B in immune cells was increased (Figs. 17, 18 and 22). Through the above series of results, it was found that the strain had an immunostimulatory effect as well as an inhibitory effect on tumor formation and tumor growth.

**Pharmaceutical use**

The pharmaceutical composition for preventing or treating cancer according to the present invention, comprising a *L. plantarum* GB104 strain or a culture product thereof as an active ingredient can increase the efficacy of cancer treatment and/or prevention. Here, a *L. plantarum* GB104 strain and a culture product thereof are the same as described above.

The cancer may be selected from the group consisting of large intestine cancer, stomach cancer, liver cancer, lung cancer, breast cancer, prostate cancer, ovarian cancer, pancreatic cancer, cervical cancer, thyroid cancer, laryngeal cancer, acute myeloid leukemia, brain tumor, neuroblastoma, retinoblastoma, head and neck cancer, salivary gland cancer, melanoma, bladder cancer, esophageal cancer, skin cancer, colorectal cancer and lymphoma, but is not limited thereto.

In the pharmaceutical composition for preventing or treating cancer according to the present invention, the active ingredient may be included in any amount (effective amount) depending on the use, formulation, blending purpose, and the like, as long as it can exhibit an anticancer activity. Typically, an effective amount of a *L. plantarum* GB104 strain will be determined within the range of 0.001% by weight to 20.0% by weight based on the total weight of the composition. Here, "effective amount" refers to the amount of an active ingredient capable of inducing an anticancer effect. Such an effective amount can be determined experimentally within the ordinary skill of those of ordinary skill in the art.

As used herein, the term "treatment" may be used to include both therapeutic treatment and prophylactic treatment. In this case, prevention may be used in the sense of alleviating or reducing the pathological condition or disease in the subject. In one embodiment, the term "treatment" includes any form of administration or application in order to treat a disease in a mammal including a human being. In addition, the term includes inhibiting or slowing a disease or progression of the disease; and it includes the meaning of restoring or repairing a damaged or missing function, thereby partially or completely alleviating the disease; or stimulating inefficient processes; or alleviating a serious disease.

As used herein, the term "efficacy" can be determined by one or more parameters, such as survival or disease-free survival over a certain period of time, such as 1 year, 5 years, or 10 years. In addition, the parameter may include inhibition of the size of at least one tumor in the subject.

Pharmacokinetic parameters such as bioavailability and underlying parameters such as clearance rate can also affect efficacy. Therefore, "enhanced efficacy" (for example, improvement in efficacy) can be attributed to enhanced pharmacokinetic parameters and enhanced efficacy, and can be measured by comparing clearance rate and tumor growth in a test animal or a human subject, or by comparing parameters such as survival, relapse rate or disease-free survival.

Here, "therapeutically effective amount" or "pharmaceutically effective amount" refers to an amount of a compound or composition effective for preventing or treating a target disease, which is an amount that is sufficient to treat the disease with a reasonable benefit/risk ratio applicable to medical treatment and that does not cause side effects. The level of the effective amount can be determined depending on factors including the health condition of the patient, the type and severity of the disease, the activity of the drug, the sensitivity to the drug, the method of administration, the time of administration, the route of administration and excretion rate, the duration of treatment, the drug to be used in combination or concurrently, and other factors well known in the medical field. In one embodiment, a therapeutically effective amount refers to an amount of a drug effective to treat cancer.

In this case, the pharmaceutical composition may further comprise a pharmaceutically acceptable carrier in addition to the active ingredient. Here, "pharmaceutically acceptable" means that the substance to be applied (prescribed) does not exhibit toxicity beyond the tolerable level without inhibiting the activity of the active ingredient. The pharmaceutically acceptable carrier may be any carrier as long as it is nontoxic material suitable for delivery to a patient. Distilled water, alcohol, fats, waxes and inert solids may be included as a carrier. In addition, a pharmacologically acceptable adjuvant (buffering agent, dispersing agent) may be included in the pharmaceutical composition.

Specifically, the cryoprotectant may be at least one selected from the group consisting of glycerol, trehalose, maltodextrin, skim milk powder, and starch. In addition, the excipient may be at least one selected from the group consisting of glucose, dextrin, and skim milk powder.

The cryoprotectant may be included in an amount of 0.01% by weight to 20% by weight, 0.01% by weight to 10% by weight based on the total weight of the composition, and specifically, the glycerol may be included in an amount of 5% by weight to 20% by weight, the trehalose may be included in an amount of 2% by weight to 10% by weight, the maltodextrin may be included in an amount of 2% by weight to 10% by weight, the skim milk powder may be included in an amount of 0.5% by weight to 2% by weight, and the starch may be included in an amount of 0.1% by weight to 1% by weight in the composition. In addition, the excipient may be included in an amount of 75% by weight to 95% by weight, or 85% by weight to 95% by weight based on the total weight of the composition.

Carriers and diluents that may be included in the pharmaceutical composition may include acacia gum, alginate, gelatin, calcium phosphate, calcium silicate, cellulose, methyl cellulose, microcrystalline cellulose, polyvinyl pyrrolidone, water, methyl hydroxybenzoate, propyl hydroxybenzoate, talc, magnesium stearate, and mineral oil. When formulated, it is prepared using diluents or excipients such as commonly used fillers, extenders, binders, wetting agents, disintegrants, and surfactants.

As long as the pharmaceutical composition according to the present invention exhibits the effects of the present invention, its dosage form, the route of administration, and method of administration are not particularly limited. The composition of the present invention may be administered to a mammal including a human being by any method. For example, it may be administered orally.

The pharmaceutical composition of the present invention may be formulated as a preparation for oral administration according to the route of administration as described above. In the case of a preparation for oral administration, the composition of the present invention may be formulated into powders, granules, tablets, pills, dragees, capsules, solutions, gels, syrups, slurries, suspensions and the like using a method known in the art.

When the pharmaceutical composition is prepared for oral administration, it may further comprise a carrier for oral administration. A carrier for oral administration may include lactose, starch, cellulose derivatives, magnesium stearate, stearic acid, and the like. Suitable stabilizers include antioxidants such as sodium bisulfite, sodium sulfite or ascorbic acid. Suitable preservatives include benzalkonium chloride, methyl- or propyl-paraben and chlorobutanol. The pharmaceutical composition of the present invention may further comprise, in addition to the above components, an anti-aggregation agent, a lubricant, a wetting agent, a sweetening agent, a flavoring agent, an emulsifier, a suspending agent, and the like.

For example, a preparation for oral administration may be obtained by combining the active ingredient with a solid excipient, milling them, adding suitable auxiliary agents, and then processing them into a mixture of granules to obtain a tablet or a dragee. Examples of suitable excipients may include sugars such as lactose, dextrose, sucrose, sorbitol, mannitol, xylitol, erythritol and maltitol, starches such as corn starch, wheat starch, rice starch and potato starch, celluloses such as cellulose, methyl cellulose, sodium carboxymethyl cellulose, and hydroxypropyl methyl cellulose, and fillers such as gelatin and polyvinylpyrrolidone. In addition, cross-linked polyvinylpyrrolidone, agar, alginic acid or sodium alginate, etc., may be added as a disintegrant, if desired.

The pharmaceutical composition of the present invention may be administered in a pharmaceutically effective amount. As used herein, the term "pharmaceutically effective amount" refers to an amount sufficient to treat or prevent the disease with a reasonable benefit/risk ratio applicable to medical treatment or prevention. The effective dose level can be determined depending on factors including the severity of the disease, the activity of the drug, the age, body weight, health and sex of the patient, the sensitivity to the drug of the patient, the time of administration of the composition of the present invention used, the route of administration and excretion rate, the duration of treatment, the drug to be used in combination or concurrently with the composition of the present invention used, and other factors well known in the medical field.

A preferred dosage of the pharmaceutical composition may be in the range of 0.01 μg/kg to 10 g/kg, or 0.01 mg/kg to 1 g/kg per day depending on the condition, body weight, sex and age of the patient, the severity of the disease, and the route of administration. Administration can be done once a day or in several divided doses. These dosages should not be construed as limiting the scope of the present invention in any aspect.

A subject to which the pharmaceutical composition can be applied (prescribed) is a mammal and a human being, and in particular, a subject is preferably a human being.

In addition to the active ingredient, the pharmaceutical composition of the present invention may further comprise any compound or natural extract whose safety has already been verified and which is known to have an anticancer activity, in order to increase or enhance an anticancer activity. When the pharmaceutical composition of the present invention is administered in combination with other therapeutic agents, it may be administered sequentially or concurrently with the conventional therapeutic agents. In addition, it may be administered in single dose or in multiple doses. It is important to administer an amount that can provide the maximum effect with the minimum amount without side effects in consideration of all the above factors.

## Use of composition comprising *L. plantarum* GB104 strain or culture product thereof

In another aspect of the present invention, there is provided the use of a pharmaceutical composition comprising a *L. plantarum* GB104 strain or a culture product thereof, for treatment of cancer. Here, the *L. plantarum* GB104 strain, cancer and treatment are the same as described above.

In another aspect of the present invention, there is provided the use of a pharmaceutical composition comprising a *L. plantarum* GB104 strain or a culture product thereof, for enhancing the therapeutic effect on cancer. Here, the *L. plantarum* GB104 strain, cancer and treatment are the same as described above.

In another aspect of the present invention, there is provided a Lactobacillus plantarum GB104 (L. plantarum GB104) strain (accession number: KCTC 14107BP), or a culture product thereof, for use in a a method for treating cancer and/or a method for enhancing the therapeutic effect, the method comprising administering the *Lactobacillus plantarum* GB104 strain or the culture product thereof to a subject. Here, the *L. plantarum* GB104 strain, cancer and treatment are the same as described above.

The subject may be a subject suffering from cancer. In addition, the subject may be a mammal, preferably a human being.

The route of administration, dosage, and frequency of administration of the *L. plantarum* GB104 strain or a culture product thereof may vary depending on the condition of the patient and the presence or absence of side effects, and thus it may be administered to a subject in various ways and amounts. The optimal administration method, dosage, and frequency of administration may be selected in an appropriate range by those of ordinary skill in the art. In addition, the above active ingredient may be administered in combination with other drugs known to have a therapeutic effect on cancer (for example, anticancer agents described above) or physiologically active substances, or may be formulated in the form of a combination preparation with other drugs.

**Mode for Carrying out the Invention**

Hereinafter, the present invention will be described in more detail by way of the following examples. However, the following examples are only for illustrating the present invention, and the scope of the present invention is not limited only to these examples.

**Example 1. Confirmation of inhibitory effect on tumor formation by treatment with *L. plantarum* GB104 strain or culture solution thereof**

The human large intestine cancer cell lines were used to confirm an inhibitory effect on tumor formation by treatment with a *L. plantarum* GB104 strain (accession number: KCTC 14107BP) or a culture solution thereof. For a strain culture supernatant (cell-free supernatant, CFS), strains in the exponential (active) growth stage cultured for about 6 hours in MRS medium were harvested, and only the supernatant, from which the strains were removed through centrifugation, was taken. The supernatant was prepared by filtering with a 0.22 $\mu$m filter.

Specifically, the human large intestine cancer cell lines HCT116 and HT-29 were each diluted in 2 mL of McCoy's 5A medium, and then dispensed to a 6-well cell culture plate at $1.5 \times 10^3$ cells/well, and stabilized for 24 hours. After 24 hours, the medium was removed and treatment with each sample together with a fresh medium was performed. General lactic acid bacteria 1 (*L. Fermentum* GB102) (accession number: KCTC14105BP) or a *L. plantarum* GB104 strain was used as a sample. The large intestine cancer cell lines were treated directly with the strain (Figs. 1 and 2), or treated with the strain in the form of strain culture supernatant (CFS) prepared by filtering (Figs. 3 and 4). When the cell lines were treated directly with the strain, treatment was performed at a concentration of $1 \times 10^7$ CFU/mL. In addition, when the cell lines were treated with the CFS, treatment was performed at a ratio of CFS to medium per well of 1:100 (20 $\mu$L/2 mL).

The medium was exchanged with a fresh medium containing the strain or strain culture supernatant at intervals of 2 to 3 days and cultured for 10 days. The cultured plate was washed twice with DPBS, and cells were fixed with 4% formalin. The fixed cells were washed twice with DPBS and stained with a 0.5% crystal violet solution for 5 minutes. Thereafter, the cells were washed with distilled water, and the colony forming ability was confirmed.

As a result, when the cell lines were treated directly with the strain, it was confirmed that tumor formation in both human large intestine cancer cell lines HCT116 and HT-29 was significantly inhibited in a group treated with a *L. plantarum* GB104 strain compared to a group treated with general lactic acid bacteria 1 (Figs. 1 and 2). In addition, even when the cell lines were treated with the strain culture supernatant, it was confirmed that tumor formation was inhibited in a group treated with a culture solution of a *L. plantarum* GB104 strain compared to a group treated with a culture solution of general lactic acid bacteria 1 (Figs. 3 and 4). Through the above results, it was confirmed that a *L. plantarum* GB104 strain was directly and indirectly involved in the inhibition of tumor formation.

**Example 2. Confirmation of cancer prevention effect of GB104 strain in animal model: CT-26 model**

The mouse-derived large intestine carcinoma CT-26 animal model was used to confirm the cancer prevention effect of a *L. plantarum* GB104 strain.

6-week-old Balb/c mice (ORIENT BIO, Korea) were purchased and acclimated for one week. At 7 weeks of age, the area around the right flank of the mice was shaved, and then Balb/c mouse-derived large intestine cancer cell line CT-26 cells were subcutaneously injected at $5 \times 10^5$ cells/100 $\mu$L per mouse to establish a tumor animal model.

General lactic acid bacteria 1 (*L. Fermentum* GB102), general lactic acid bacteria 2 (*L. Fermentum* GB103) (accession number: KCTC14106BP) or a *L. plantarum* GB104 strain was orally administered to the animal model at $1 \times 10^9$ CFU/mouse every day starting from the time of tumor cell injection continuing until just prior to the end of the test (Fig. 5). The tumor size was measured using a digital calliper, and the tumor volume was calculated using the following [Equation

1].

[Equation 1]

$$\text{Tumor volume (mm}^3\text{)} = (\text{short axis (width)}^2 \times \text{long axis (length)})/2$$

As a result, in the case of a group administered with general lactic acid bacteria 1 (*L. Fermentum* GB102) or general lactic acid bacteria 2 (*L. Fermentum* GB103), no significant difference in the tumor growth rate was observed compared to the negative control group. On the other hand, in the case of a group administered with a *L. plantarum* GB104 strain, it was confirmed that the tumor growth rate was significantly inhibited compared to the negative control group (Control) (Figs. 6 and 7). Through the above results, it was confirmed that administration with a *L. plantarum* GB104 strain showed the tumor prevention effect on tumor formation.

**Example 3. Confirmation of cancer treatment effect of GB104 strain in animal model: CT-26 model**

The mouse-derived large intestine carcinoma CT-26 animal model was used to confirm the tumor treatment effect by administration with a *L. plantarum* GB104 strain.

A tumor animal model was established in the same manner as in Example 2, and the formed tumor was measured. On the 5th day of tumor cell injection, only mice with a tumor size within the range of 30 to 50 mm$^3$ were selected, and each group was randomly set. General lactic acid bacteria 3 (*L. Helveticus* MG585) (accession number: KCTC14110BP) or a *L. plantarum* GB104 strain was used as a sample and was orally administered to the animal model at $1 \times 10^9$ CFU/mouse every day starting from the 6th day continuing until just prior to the end of the test (Fig. 8).

As a result, in the case of a group administered with general lactic acid bacteria 3 (*L. Helveticus* MG585), no significant difference in the tumor growth rate was observed compared to the negative control group. On the other hand, in the case of a group administered with a *L. plantarum* GB104 strain, it was confirmed by measuring the tumor size and weight that the tumor growth rate was significantly inhibited compared to the negative control group (Figs. 9 to 11). Through the above results, it was confirmed that administration with a *L. plantarum* GB104 strain showed the tumor treatment effect after tumor formation.

**Example 4. Confirmation of cancer treatment effect of GB104 strain in animal model: MC-38 model**

The mouse-derived large intestine carcinoma MC-38 animal model was used to confirm the tumor treatment effect by administration with a *L. plantarum* GB104 strain.

6-week-old C57BL/6 mice (ORIENT BIO, Korea) were purchased and acclimated for one week. At 7 weeks of age, the area around the right flank of the mice was shaved, and then C57BL/6 mouse-derived large intestine cancer cell line MC-38 cells were subcutaneously injected at $5 \times 10^5$ cells/100 μL per mouse to establish a tumor animal model. The tumor size was measured and calculated in the same manner as in Example 2.

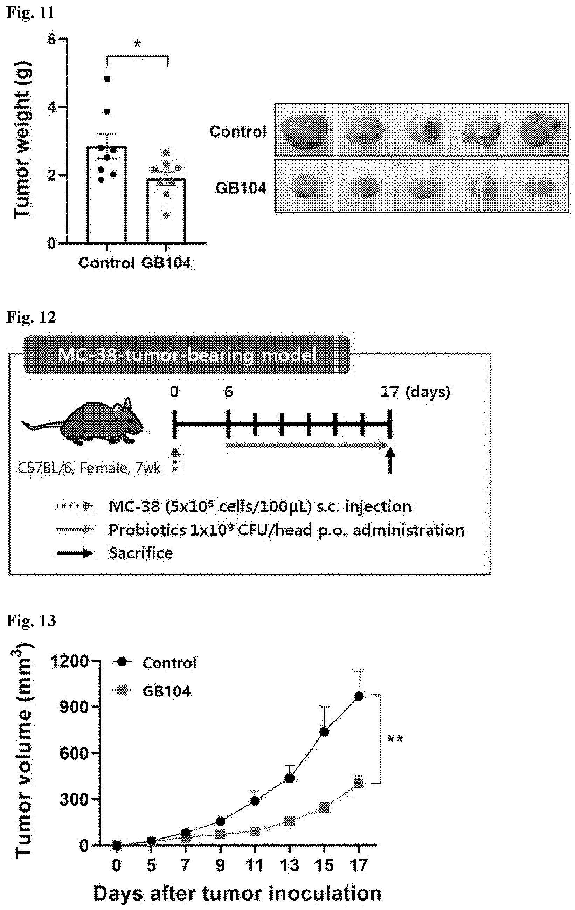

On the 5th day of tumor cell injection, only mice with a tumor size within the range of 30 to 50 mm$^3$ were selected, and each group was randomly set. A *L. plantarum* GB104 strain was orally administered to the animal model at $1 \times 10^9$ CFU/mouse every day starting from the 6th day continuing until just prior to the end of the test (Fig. 12).

As a result, in the case of a group administered with a *L. plantarum* GB104 strain, it was confirmed by measuring the tumor size and weight that the tumor growth rate was significantly inhibited compared to the negative control group (Figs. 13 to 15). Through the above results, it was confirmed that administration with a *L. plantarum* GB104 strain showed the tumor treatment effect after tumor formation.

**Example 5. Confirmation of changes in immune cells in early stage tumor tissue** and **peripheral blood after administration with GB104 strain: MC-38 model**

The mouse-derived large intestine carcinoma MC-38 animal model was used to confirm the changes in immune cells in the early stage tumor tissue and the early stage peripheral blood by administration with a *L. plantarum* GB104 strain.

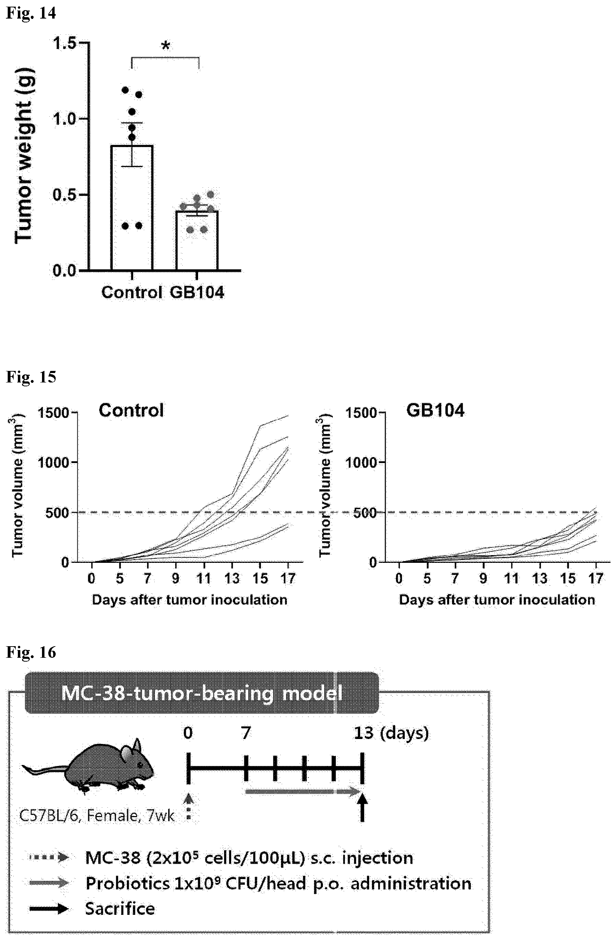

Specifically, a tumor model was established in the same manner as in Example 4 by subcutaneously injecting C57BL/6 mouse-derived large intestine cancer cell line MC-38 cells at $2 \times 10^5$ cells/100 μL per mouse, and the mice were orally administered with a *L. plantarum* GB104 strain at $1 \times 10^9$ CFU/mouse every day starting from the 7th day. 6 days after the start of administration (13th day), tumor tissues and blood were collected from the mice, and immune cells were isolated (Fig. 16). The isolated immune cells were stained with fluorescent antibodies that bind to each immune cell marker and then analyzed using FACSymphony equipment. Information of the antibodies is shown in Table 1.

[Table 1]

| Antibody | Manufacturer | Cat. NO |
|---|---|---|
| anti-CD8a-Pacific Blue | Biolegend | 100725 |
| anti-CD45-BV711 | Biolegend | 103147 |
| anti-CD3-FITC | Biolegend | 100204 |
| anti-NK-1.1-PE | Biolegend | 108708 |
| anti-Granzyme B-PE-Cy7 | eBioscience | 25-8898-82 |
| anti-IFN-γ-APC | Biolegend | 505810 |
| anti-CD25-PE | Biolegend | 101904 |
| anti-CD4-PE-Cy7 | Biolegend | 100422 |
| anti-Foxp3-APC | eBioscience | 17-5773-82 |
| anti-CD11b-Pacific Blue | Biolegend | 101224 |
| anti-F4/80-FITC | Biolegend | 123108 |
| anti-CD206-PE | Biolegend | 141706 |
| anti-CD45-APC | Biolegend | 103112 |

As a result, in the case of immune cells in the early stage tumor tissue, granzyme B secreted from NK cells was significantly increased in a group administered with a *L. plantarum* GB104 strain compared to the negative control group. In addition, the ratio of regulatory T cells (Tregs cells), which are known to enable tumor immune evasion by inhibiting tumor specific immune responses and establishing an immunosuppressive tumor microenvironment, was significantly reduced in a group administered with a *L. plantarum* GB104 strain (Fig. 17).

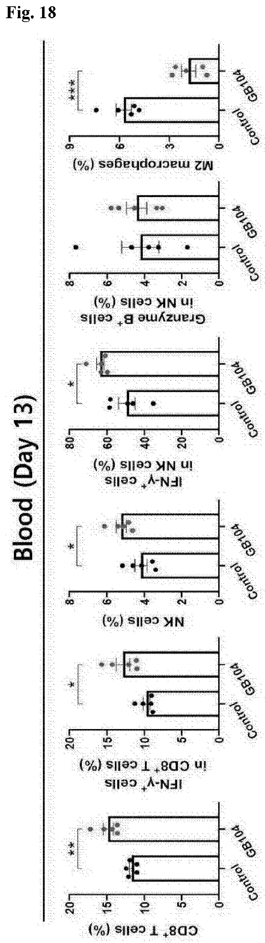

In the case of immune cells in the peripheral blood, the ratio of cytotoxic T cells (CD8$^+$ T cells) and natural killer cells (NK cells), which are known to be immune cells that directly inhibit cancer growth, was significantly increased in a group administered with a *L. plantarum* GB104 strain compared to the negative control group. In addition, IFN-γ secreted from these cells was also significantly increased. In addition, it was confirmed that M2 macrophages, which are known to help cancer cell proliferation, were significantly reduced in a group administered with a *L. plantarum* GB104 strain (Fig. 18).

**Example 6. Confirmation of changes in immune cells in late stage tumor tissue after administration with GB104 strain: MC-38 model**

The mouse-derived large intestine carcinoma MC-38 animal model was used to confirm the changes in immune cells in the late stage tumor tissue by administration with a *L. plantarum* GB104 strain.

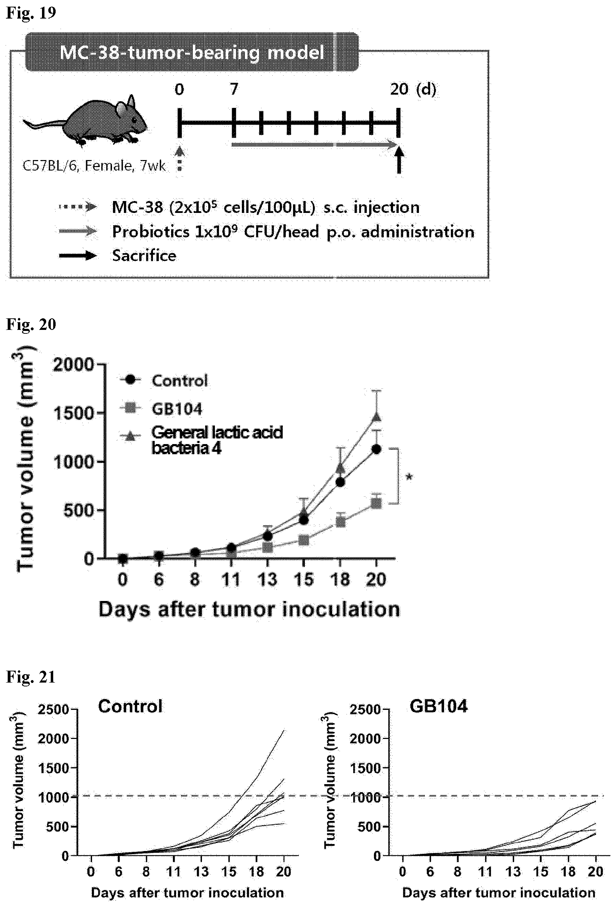

Specifically, a tumor model was established in the same manner as in Example 5, and the mice were orally administered with general lactic acid bacteria 4 (*L. plantarum* GBCC_f0057; GI Biome's own isolated and identified bacteria) or a *L. plantarum* GB104 strain at $1 \times 10^9$ CFU/mouse every day starting from the 7$^{th}$ day. 13 days after administration (20$^{th}$ day), the test was terminated, and tumor tissues were collected from the mice, and immune cells were isolated (Fig. 19). The isolated immune cells were stained with fluorescent antibodies in the same manner as in Example 5 and then analyzed using FACSymphony equipment. The antibodies shown in Table 1 were used.

As a result, tumors of a similar size to those of the negative control group were observed in a group administered with general lactic acid bacteria 4 (*L. plantarum* GBCC_f0057) compared to the negative control group, whereas the size of the tumor was significantly reduced in a group administered with a *L. plantarum* GB104 strain compared to the negative control group (Figs. 20 and 21).

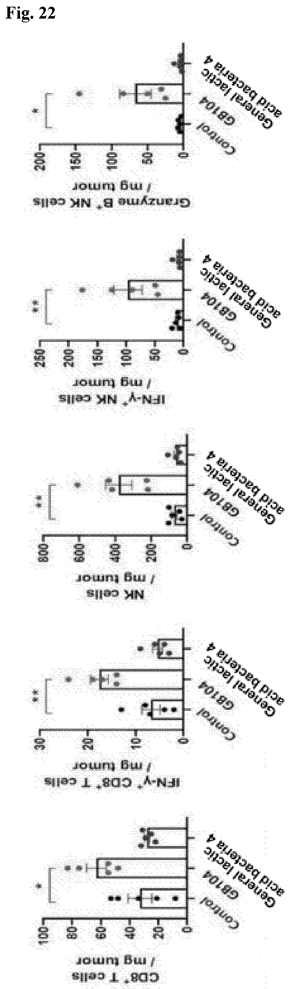

In addition, it was confirmed that the number of cytotoxic T cells (CD8$^+$ T cells) and natural killer cells (NK cells) in the late stage tumor tissue was significantly increased in a group administered with a *L. plantarum* GB 104 strain compared to the tumor tissue collected from the negative control group. In addition, it was confirmed that IFN-γ and granzyme B secreted from these cells were also significantly increased. On the other hand, it was confirmed that a group administered with general lactic acid bacteria 4 (*L. plantarum* GBCC_f0057) showed the results similar to those of the negative control group (Fig. 22).

Through the above results, it was confirmed that the number of immune cells involved in anti-tumor immunity was increased by administration with a *L. plantarum* GB 104 strain, and it was confirmed that the changes in immune cells were associated with the anticancer activity.

[Accession number]

Name of Depositary authority: Korean Collection for Type Cultures (KCTC), Korea Research Institute of Bioscience and Biotechnology
Accession Number: KCTC14107BP
Deposit Date: January 14, 2020

## Claims

1. A pharmaceutical composition for use in preventing or treating cancer, comprising a *Lactobacillus plantarum* GB104 (*L. plantarum* GB104) strain (accession number: KCTC 14107BP) or a culture product thereof as an active ingredient.

2. The pharmaceutical composition for use in preventing or treating cancer according to claim 1, wherein the strain or culture product thereof exhibits an inhibitory effect on tumor formation.

3. The pharmaceutical composition for use in preventing or treating cancer according to claim 1, wherein the strain exhibits an immunostimulatory effect.

4. The pharmaceutical composition for use in preventing or treating cancer according to claim 1, wherein the cancer is any one selected from the group consisting of large intestine cancer, stomach cancer, liver cancer, lung cancer, breast cancer, prostate cancer, ovarian cancer, pancreatic cancer, cervical cancer, thyroid cancer, laryngeal cancer, acute myeloid leukemia, brain tumor, neuroblastoma, retinoblastoma, head and neck cancer, salivary gland cancer, melanoma, bladder cancer, esophageal cancer, skin cancer, colorectal cancer and lymphoma.

5. A *Lactobacillus plantarum* GB104 (*L. plantarum* GB104) strain (accession number: KCTC 14107BP), or a culture product thereof, for use in a method for preventing or treating cancer, the method comprising administering the *Lactobacillus plantarum* GB104 (*L. plantarum* GB104) strain (accession number: KCTC 14107BP) or the culture product thereof to a subject other than a human being.

## Patentansprüche

1. Pharmazeutische Zusammensetzung zur Verwendung beim Vorbeugen oder Behandeln von Krebs, umfassend einen *Lactobacillus-plantarum*-GB104(*L.-plantarum*-GB104)-Stamm (Zugangsnummer: KCTC 14107BP) oder ein Kulturprodukt davon als einen Wirkstoff.

2. Pharmazeutische Zusammensetzung zur Verwendung beim Vorbeugen oder Behandeln von Krebs nach Anspruch 1, wobei der Stamm oder das Kulturprodukt davon eine hemmende Wirkung auf die Tumorbildung zeigt.

3. Pharmazeutische Zusammensetzung zur Verwendung beim Vorbeugen oder Behandeln von Krebs nach Anspruch 1, wobei der Stamm eine immunstimulierende Wirkung zeigt.

4. Pharmazeutische Zusammensetzung zur Verwendung beim Vorbeugen oder Behandeln von Krebs nach Anspruch 1, wobei es sich bei dem Krebs um einen beliebigen ausgewählt aus der Gruppe bestehend aus Dickdarmkrebs, Magenkrebs, Leberkrebs, Lungenkrebs, Brustkrebs, Prostatakrebs, Eierstockkrebs, Bauchspeicheldrüsenkrebs, Gebärmutterhalskrebs, Schilddrüsenkrebs, Kehlkopfkrebs, akuter myeloischer Leukämie, Gehirntumor, Neuroblastom, Retinoblastom, Kopf- und Halskrebs, Speicheldrüsenkrebs, Melanom, Blasenkrebs, Speiseröhrenkrebs, Hautkrebs, Kolorektalkrebs und Lymphom handelt.

5. *Lactobacillus-plantarum*-GB104(*L.-plantarum*-GB104)-Stamm (Zugangsnummer: KCTC 14107BP) oder Kulturprodukt davon zur Verwendung in einem Verfahren zum Vorbeugen oder Behandeln von Krebs, wobei das Verfahren Verabreichen des *Lactobacillus-plantarum*-GB104(*L.-plantarum*-GB104)-Stamms (Zugangsnummer: KCTC 14107BP) oder des Kulturprodukts davon an ein anderes Subjekt als einen Menschen umfasst.

**Revendications**

1. Composition pharmaceutique destinée à être utilisée dans la prévention ou le traitement du cancer, comprenant une souche *Lactobacillus plantarum* GB104 (*L. plantarum* GB104) (numéro d'accès : KCTC 14107BP) ou un produit de culture de celle-ci en tant qu'ingrédient actif.

2. Composition pharmaceutique destinée à être utilisée dans la prévention ou le traitement du cancer selon la revendication 1, dans laquelle la souche ou le produit de culture de celle-ci présente un effet inhibiteur sur la formation de tumeurs.

3. Composition pharmaceutique destinée à être utilisée dans la prévention ou le traitement du cancer selon la revendication 1, dans laquelle la souche présente un effet immunostimulateur.

4. Composition pharmaceutique destinée à être utilisée dans la prévention ou le traitement du cancer selon la revendication 1, ledit cancer étant l'un quelconque choisi dans le groupe constitué par le cancer du gros intestin, le cancer de l'estomac, le cancer du foie, le cancer du poumon, le cancer du sein, le cancer de la prostate, le cancer de l'ovaire, le cancer du pancréas, le cancer du col de l'utérus, le cancer de la thyroïde, le cancer du larynx, la leucémie myéloïde aiguë, la tumeur cérébrale, le neuroblastome, le rétinoblastome, le cancer de la tête et du cou, le cancer des glandes salivaires, le mélanome, le cancer de la vessie, le cancer de l'œsophage, le cancer de la peau, le cancer colorectal et le lymphome.

5. Souche *Lactobacillus plantarum* GB104 (*L. plantarum* GB104) (numéro d'accession : KCTC 14107BP), ou produit de culture de celle-ci, destiné(e) à être utilisé(e) dans un procédé de prévention ou de traitement du cancer, le procédé comprenant l'administration de la souche *Lactobacillus plantarum* GB104 *(L. plantarum* GB104) (numéro d'accession : KCTC 14107BP) ou du produit de culture de celle-ci à un sujet autre qu'un être humain.

**Fig. 1**

Probiotics (1x10$^7$ CFU/mL)

**Fig. 2**

**Fig. 3**

* CFS (cell-free supernatants)

Fig. 4

Fig. 5

Fig. 6

Fig. 7

Fig. 8

Fig. 9

Fig. 10

Fig. 11

Fig. 12

MC-38-tumor-bearing model

C57BL/6, Female, 7wk

0    6    17 (days)

····▶ MC-38 (5x10$^5$ cells/100μL) s.c. injection
——▶ Probiotics 1x10$^9$ CFU/head p.o. administration
——▶ Sacrifice

Fig. 13

Fig. 14

Fig. 15

Fig. 16

**Fig. 17**

**Fig. 18**

Fig. 19

Fig. 20

Fig. 21

**Fig. 22**

**EP 4 316 499 B1**

### Patent documents cited in the description

- KR 1020150068061
- KR 101287120
- KR 20130132398